# EUROPEAN PATENT APPLICATION

(11) **EP 0 724 158 A1**
(43) Date of publication of application: **31.07.1996**
(21) Application number: 95926015.9
(22) Date of filing: 24.07.1995
(51) Int. Cl.: G01N 33/564

(54) **METHOD OF IMMUNOASSAYING AUTOANTIBODY**

(30) Priority: 25.07.1994 JP 172726/94
(71) Applicant: KANEGAFUCHI KAGAKU KOGYO KABUSHIKI KAISHA, Kita-ku Osaka-shi Osaka 530 (JP)
(72) Inventor: YABUKI, Tetsuro, Kobe-shi Hyogo-ken 654-01 (JP)
(74) Representative: Mallalieu, Catherine Louise
(86) International application number: JP9501466
(87) International publication number: WO9603654

(57) **Abstract**

A method for immunologically measuring antibodies specific to systemic lupus erythematosus (SLE) and a kit for the measurement are provided. The amount of antibody binding to a negatively charged substance through electrostatic affinity can be derived by allowing a humor to react with the negatively charged substance in at least three kinds of buffer solutions having different ion intensities so as to measure the binding amount of the antibody to the negatively charged substance in each buffer solution, and evaluating a difference between the minimum value of binding amounts and the maximum value of binding amounts at ion intensities lower than the ion intensity corresponding to the minimum value. The measurement method and the measurement kit enable a specific and easy measurement of autoantibodies binding to an antigen through electrostatic affinity, which are considered to be closely related to the pathosis of SLE, and a more accurate diagnosis of SLE.

## Description

### Field of the Invention

The present invention relates to a method for immunologically measuring antibodies characteristically found in systemic lupus erythematosus (SLE).

### Prior Art

Autoimmune diseases are diseases in which, owing to malfunctions of the immune system, humoral or cellular immune responses against one's own cells or tissues occur, whereby antibodies (autoantibodies) reacting with one's own tissues appear. In the case of systemic lupus erythematosus (SLE), a typical autoimmune disease, autoantibodies such as antibodies against nucleus components of cells, in particular those (anti-DNA antibodies) against deoxyribonucleic acid (DNA), and antibodies against cell membrane components, in particular those (anti-CL antibodies) against cardiolipin (CL), appear in humors; such autoantibodies are known to be related to pathosis. Based on such information, the measurement of such autoantibodies has been conducted in clinical examinations.

To measure autoantibodies, methods considering the affinity of antibodies against antigens are known, e.g., a method (A.J. G. Swaak, Protides of Biological Fluids, vol. 33, edited by M. Peeters, Pergamon Press, New York, pp. 317-320 (1985)) which evaluates a ratio between an anti-DNA antibody titer obtained by ammonium sulfate fractionation and an anti-DNA antibody titer obtained by polyethyleneglycol precipitation, and a method (H. Mcgrath. Jr., Arthritis and Rheumatism, vol. 28, No. 4, pp.425-430 (1985)) in which DNA is allowed to react, as an antigen, with an anti-DNA antibody, whereafter a large amount of DNA or salt is added, and the affinity of the anti-DNA antibody for the antigen is measured based on the dissociated amount of DNA or anti-DNA antibody, are known.

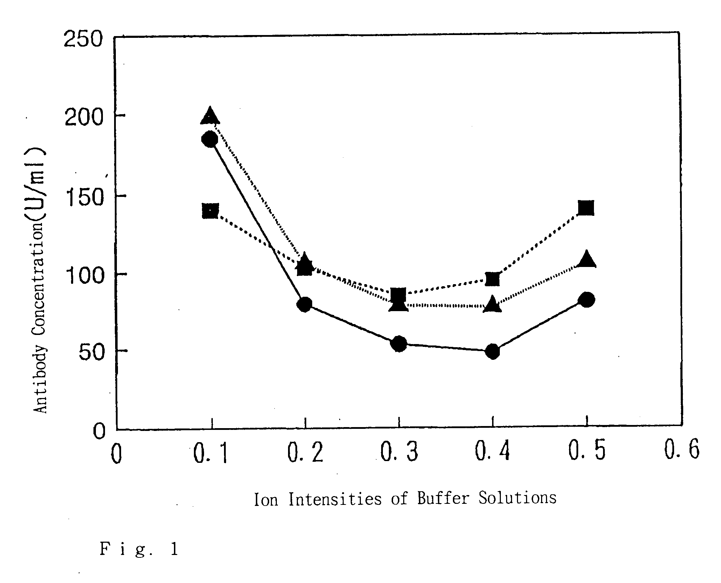

In both of the above methods, after the formation of a complex of an antigen and an autoantibody, the binding equilibrium between the antigen and the antibody is broken with high salt concentration, extreme change in pH, excessive antigen treatment, or the like, then the dissociated autoantibodies are measured. However, such methods are subject to overestimation errors, higher than the affinity under physiological conditions, due to the treatment for dissociating the antigen-antibody complex (e.g., high salt concentration treatment) because, in the case where hydrophobic bonds or hydrogen bonds are involved in the binding between the antigen and the autoantibody, such bonds become stronger, as the ambient salt concentration increases. For example, Figure **1** shows the relationship between the amount of autoantibody in the sera of patients 1 (●), 2 (■), and 3 (▲) with SLE and the ion intensities of a buffer solution. The amount of antibody binding to the antigen increases when the ambient ion intensity exceeds a certain ion intensity (critical point). As a result, there is also a danger of ruining the specific antigen-antibody binding conditions, thereby leading to misevaluation of the exact binding amount.

In recent years, reports have been made indicating that not only the kind, amount, and antibody titer of an autoantibody, but also that the binding strength between the antibody and antigen is also related to pathosis. Therefore, the correlation between an autoantibody's affinity to an antigen and pathosis is drawing attention. Accordingly, there is desired a method for antibody measurement which can accurately indicate pathosis. With a view to solving these problems, a method (Japanese Patent Application No. 1-290964) was devised, in which the binding activity between a given DNA, which is an antigen, and an anti-DNA antibody is measured under the conditions of two kinds of buffer solutions having different salt concentrations, so as to derive a ratio between the anti-DNA antibody binding activities measured under the respective conditions. However, according to this method, the measurement values of the autoantibody and pathosis are not sufficiently correlated, and the amount of autoantibody does not necessarily reflect pathosis. Accordingly, the problem of antibody measurement that reflects pathosis has not been solved yet.

### Disclosure of the Invention

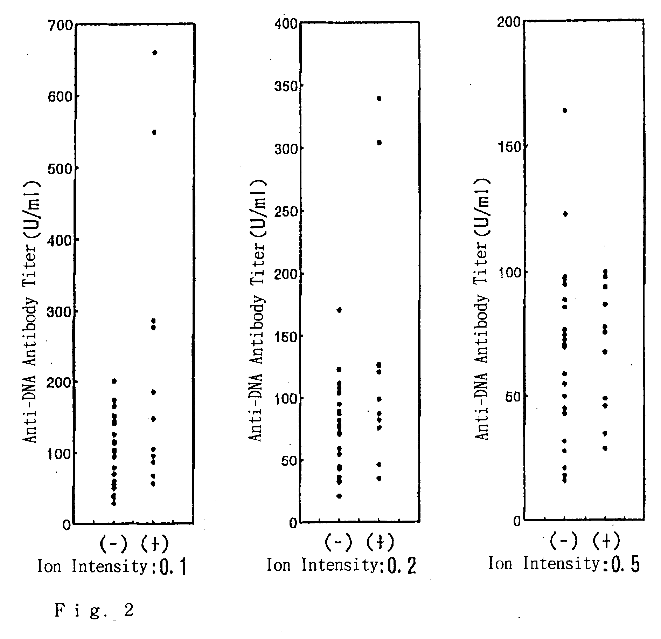

The present inventor has conducted a great deal of research to discover that not all the autoantibodies (e.g., anti-DNA antibodies and anti-CL antibodies), whose relationship with the pathosis of SLE has been pointed out, or all the autoantibodies having high affinities with antigens are related to pathosis, but rather that those autoantibodies binding to antigens mainly through electrostatic affinity, and in particular those autoantibodies which bind to negatively charged substances through electrostatic affinity, are related to pathosis (in particular kidney disorders). For example, the relationship between the presence/absence (indicated as + or -) of kidney disorders and anti-DNA antibody titers shown in Figure **2** reveals no difference in the anti-DNA antibody titers of a normal person and a patient when the ion intensity exceeds 0.5 (i.e., under conditions which substantially inhibit any binding through electrostatic affinity), indicating no relationship with pathosis.

Accordingly, a method for measuring an autoantibody has been discovered, in which a humor and a negatively charged substance are allowed to react in at least three kinds of buffer solutions having different ion intensities under conditions which do not affect the electrostatic affinity so as to measure the binding amount of an autoantibody to the negatively charged substance in each buffer solution; a difference between the minimum value of binding amounts and the maximum value of binding amounts at ion intensities lower than the ion intensity corresponding to the minimum binding amounts, thereby determining the amount of autoantibody which binds to the negatively charged substance through electrostatic affinity. Thus, the present invention is accomplished. Since the method of the present invention makes it possible to eliminate any non-specific binding between antigen-antibody by conducting a measurement at a certain ion intensity or less, it enables the measurement of the autoantibody amount corresponding to pathosis. Therefore, the present invention provides a method for measuring autoantibodies that are correlated with pathosis, which could not be attained by the method described in Japanese Patent Application No. 1-290964.

Accordingly, the present invention provides a method which includes the steps of allowing a humor to react with a negatively charged substance in at least three kinds of buffer solutions having different ion intensities to measure the binding amount of the antibody included in the humor to the negatively charged substance in each buffer solution, and a step of determining the amount of antibody binding to the negatively charged substance by evaluating a difference between the minimum value of binding amounts and the maximum value of binding amounts at ion intensities lower than the ion intensity corresponding to the minimum value.

In a preferred embodiment, the ion intensities of the buffer solutions are about 0.50 or less, and the difference between the ion intensities is about 0.01 to about 0.50.

In a preferred embodiment, the humor is blood, plasma, serum, ascites, lymph, intra-articular fluid, cerebrospinal fluid, or a fraction component obtained therefrom.

In a preferred embodiment, the negatively charged substance is DNA or cardiolipin (CL) or a compound containing one, two, or more of a sulfate ester group, a sulfonic acid group, a carboxyl group, or a phosphate ester group.

In a preferred embodiment, the salt used for the buffer solutions is NaCl, KCl, or CaCl₂.

In a preferred embodiment, the measurement of the binding amount is conducted by enzyme immunoassay.

The present invention also provides a measurement kit for performing the above measurement method, which includes at least three kinds of buffer solutions having different ion intensities.

The present invention also provides a method for diagnosing systemic lupus erythematosus (SLE) which includes the steps of allowing a humor to react with a negatively charged substance in at least three kinds of buffer solutions having different ion intensities to measure the binding amounts of the autoantibody to the negatively charged substance in the at least three buffer solutions, and evaluating the amount of the autoantibody binding to the negatively charged substance by deriving a difference between the minimum value of binding amounts and the maximum value of binding amounts at ion intensities lower than the ion intensity corresponding to the minimum value.

### Brief Description of the Drawings

Figure **1** is a diagram showing the relationship between ion intensities of buffer solutions and amounts of an antibody binding to an antigen in the serum of a patient with SLE in an antigen-antibody reaction.

Figure **2** is a diagram showing the relationship between ion intensities of buffer solutions and anti-DNA antibody titers of a normal person (-) and a patient with SLE (+) obtained by using a conventional method.

Figure **3** is a diagram showing the results of EAA measurement, and results of measurement using a common anti-DNA antibody measurement kit, of plasma of a normal person (-) and that of a patient with SLE (+).

### Embodiments of the Invention

Hereinafter, the present invention will be described in detail.

Autoantibodies which can be measured by the measurement method of the present invention include antinuclear antibodies, anti-DNA antibodies, anti-CL antibodies, antiglomerular basement membrane antibodies, anti-heparan sulfate antibodies, and the like. Particularly preferable are anti-DNA antibodies and anti-CL antibodies specific to systemic lupus erythematosus (SLE).

As humors to be used for the measurement method of the present invention, any of blood, plasma, serum, ascites, lymph, intra-articular fluid, cerebrospinal fluid, or a fraction component obtained by fractionating these by gel chromatography, ammonium sulfate fractionation, etc, or other fluid components from an organism can be used.

Negatively charged substances which can be used for the measurement method of the present invention include substances from an organism conventionally used for the measurement of autoantibodies of SLE, such as DNA and CL, or a compound containing one, two, or more of functional groups capable of being negatively charged in the vicinity of a neutral pH, e.g., a sulfate ester group, a sulfonic acid group, a carboxyl group, or a phosphate ester group.

Salts which can be used for the above buffer solutions are strong electrolytes which completely ionize in water. Particularly preferable are NaCl, KCl, and CaCl₂.

Ion intensity buffer solutions which can be used for the present invention have intensities which do not allow non-specific binding between negatively charged substance-autoantibody due to hydrophobic bonds and the like, which do not occur under physiological conditions. Preferably the ion intensity is about 0.50 or less. The difference in ion intensity between the respective ion intensity buffer solutions is about 0.01 to about 0.5, and preferably about 0.1 to about 0.3.

As the method of measuring antibodies which can be used for the present invention, known immunological measurement methods such as a fluorescent antibody technique, a passive hemagglutination, a precipitin reaction, a radioimmunoassay, an enzyme immunoassay (EIA method) and the like are available. An EIA method, in particular an enzyme-linked immunosorbent assay (ELISA method), is preferable in terms of convenience, safety, and quantification.

By using the EIA method, antibody titers can be measured as follows, for example. First, a negatively charged substance such as DNA or CL, used as an antigen, is immobilized to a solid phase, whereafter a treatment for preventing non-specific absorption to the antigen is performed using bovine serum albumin or the like. Next, a low ion-intensity buffer solution and a high ion-intensity buffer solution are prepared, to each of which a sample (humor) is added. Thus, an antigen-antibody reaction is effected. After the reaction is finished, the antibody bound to the solid phase is further allowed to react with an enzyme-labeled antibody. By measuring the activity of the enzyme bound to the solid phase, the antibody amount can be obtained. As the labeling enzyme, horseradish peroxidase, alkaline phosphatase, β-galactosidase, or the like can be used.

From the antibody amounts measured in at least three kinds of buffer solutions, a critical point indicating the minimum value among the measured samples is derived. By evaluating a difference between the antibody amount at the critical point and the maximum value of the antibody amounts at ion intensities lower than the critical point, the autoantibody amount can be derived. In general, the ion intensity at the critical point is not constant due to individual differences, pathological differences, and the like, but the ion intensity is likely to be about 0.2 to about 0.5, and in particular about 0.3 to about 0.4, as shown in Figure **1**.

The measurement kit for performing the method for measuring autoantibodies includes at least three kinds of buffer solutions having different ion intensities and, for example, includes a microtiter plate to which an antigen is immobilized, an enzyme-labeled antibody, a solution containing a substrate for the enzyme, and samples for the calibration curve having known antibody titers.

By measuring the autoantibody amount in a humor with the method of measuring autoantibodies according to the present invention, SLE may be diagnosed with greater accuracy (that is, markedly decreasing false positive results and false negative results).

### Examples

Next, the present invention is specifically described by way of examples.

### [Example 1]

A hundred µl of Poly-L-Lysine (10 µg/ml; manufactured by Sigma), diluted with Tris buffer (0.15 M Tris-HCl, pH 7.6), was placed into each well of a 96-well microtiter plate (manufactured by Nunc), and left at room temperature for 30 minutes. Thereafter, the plate was washed three times with a washing solution (0.015 M Tris-HCl containing 0.05% Tween 20 + 0.135 M NaCl, pH 7.6), and precoated. Then, 100 µl of double-stranded DNA (10 µg/ml) diluted with Tris buffer was placed into each well, and was allowed to stand at room temperature for 30 minutes. The plate was then washed three times with a washing solution, thereby immobilizing the antigen.

This double-stranded DNA was obtained by treating DNA derived from a bovine thymus (manufactured by Boehringer Mannheim Gmbh) with an enzyme (S1 nuclease (1 U/ml, 0.5 mM ZnSO₄, 0.1 M acetate buffer, pH 6.0; manufactured by SANKYO) at 37°C for 30 minutes).

Three hundred µl of Tris buffer containing 1% bovine serum albumin (manufactured by Sigma; hereinafter referred to as "BSA") was placed in each well of the plate to which the antigen had been immobilized, and left at room temperature for 1 hour. Thus, BSA was allowed to be adsorbed to portions other than portions to which the DNA had been immobilized, thus conducting a treatment for preventing antibodies other than antibodies binding to the double-stranded DNA from being adsorbed in a non-specific manner. Buffer solutions having ion intensities of 0.2, 0.3, and 0.5, respectively (1% BSA, 0.15 M Tris-HCl, pH 7.6; containing 0.2, 0.3, and 0.5 M of NaCl, respectively), were prepared. A hundred µl each of plasma from 5 patients with SLE, diluted by 400 times using these buffer solutions, was placed into two wells, and was allowed to stand at 4°C overnight. After the reaction had finished, the plate was washed three times with 200 µl of a washing solution. A hundred µl of peroxidase-labeled rabbit anti-human IgG(Fc) antibody (manufactured by Cappel), diluted by 2000 times with Tris buffer containing 1% BSA, was placed into each well, and allowed to react at room temperature for 1 hour.

After the immunological reaction had finished, the plate was washed three times with 200 µl of a washing solution. A hundred µl of a substrate solution (0.04% o-phenylenediamine, 0.014% H₂O₂, 0.15 M citrate-phosphate buffer, pH 4.2) was placed in the plate, and allowed to stand at room temperature for 4 minutes. Then, the color developing reaction was terminated by adding 100 µl of 2.5 M H₂SO₄, and the absorption at 492 nm was measured with a microplate reader (manufactured by BioRad).

A standard serum having an anti-double-stranded DNA antibody titer of 100 U/ml was diluted using Tris buffer containing 1% BSA, and the antibody titers which bound to the antigen in the respective ion intensity buffer solutions under the respective circumstances were obtained based on the produced calibration curve (0 to 100 U/ml).

A difference between the antibody titer at the critical point in each sample thus obtained and the maximum antibody titer at ion intensities lower than the critical point was derived, and antibodies which bound to the double-stranded DNA through electrostatic affinity (Electro-static Affinity Antibody: EAA) were detected.

The results obtained are shown in Table 1 in comparison with those of a conventional method. The anti-DNA antibody titers used for the conventional method were obtained by "Recombigen ELISA anti-ds DNA kit" commercially available from Nihon DPC.

**Table 1**

| Comparison with conventional method | | |
|---|---|---|
| SLE patients | Conventional method Anti-DNA antibody titer (IU/ml) | Present invention EAA (U/ml) |
| Patient A | 92 | 34 |
| Patient B | 44 | 28 |
| Patient C | 43 | 16 |
| Patient D | 26 | 20 |
| Patient E | 24 | 16 |

As shown in Table 1, antibodies (EAA) binding to DNA through electrostatic affinity were successfully measured by using double-stranded DNA as a negatively charged substance. Since the EAA measured by the method of the present invention is distinct from the anti-DNA antibody titers obtained by the conventional method, which is based only on the intensity of affinity for DNA, the inequality relationship between some of the resultant values was different.

### [Example 2]

A hundred µl of Poly-L-Lysine (10 µg/ml; manufactured by Sigma), diluted with Tris buffer (0.15 M Tris-HCl, pH 7.6), was placed into each well of a 96-well microtiter plate (manufactured by Nunc), and left at room temperature for 30 minutes. Thereafter, the plate was washed three times with a washing solution (0.015 M Tris-HCl containing 0.05% Tween 20 + 0.135 M NaCl, pH 7.6), and precoated. Then, 100 µl of double-stranded DNA (10 µg/ml) diluted with Tris buffer was placed into each well, and was allowed to stand at room temperature for 30 minutes. The plate was then washed three times with a washing solution, thereby immobilizing the antigen.

This double-stranded DNA was obtained by treating DNA derived from a bovine thymus (manufactured by Boehringer Mannheim Gmbh) with an enzyme (S1 nuclease (1 U/ml, 0.5 mM ZnSO₄, 0.1 M acetate buffer, pH 6.0; manufactured by SANKYO) at 37°C for 30 minutes).

Three hundred µl of Tris buffer containing 1% BSA (manufactured by Sigma) was placed in each well of the plate to which the antigen had been immobilized, and left at room temperature for 1 hour. Thus, BSA was allowed to be adsorbed to portions other than portions to which the DNA had been immobilized, thus conducting a treatment for preventing antibodies other than antibodies binding to the double-stranded DNA from being adsorbed in a non-specific manner. Buffer solutions having respective intensities (1% BSA, 0.15 M Tris-HCl, pH 7.6; containing 0.2, 0.3, and 0.5 M of NaCl, respectively), were prepared. A hundred µl each of plasma from 5 patients with SLE (a group of 12 samples (+) with albuminuria and a group of 18 samples (-) without albuminuria), diluted by 400 times using these buffer solutions, was placed into two wells, and was allowed to stand at 4°C overnight. After the reaction had finished, the plate was washed three times with 200 µl of a washing solution. A hundred µl of peroxidase-labeled rabbit anti-human IgG(Fc) antibody (manufactured by Cappel), diluted by 2000 times with Tris buffer containing 1% BSA, was placed into each well, and allowed to react at room temperature for 1 hour.

After the immunological reaction had finished, the plate was washed three times with 200 µl of a washing solution. A hundred µl of a substrate solution (0.04% o-phenylenediamine, 0.014% H₂O₂, 0.15 M citrate-phosphate buffer, pH 4.2) was placed in the plate, and allowed to stand at room temperature for 4 minutes. Then, the color developing reaction was terminated by adding 100 µl of 2.5 M H₂SO₄, and the absorption at 492 nm was measured with a microplate reader (manufactured by BioRad).

A standard serum having an anti-double-stranded DNA antibody titer of 100 U/ml was diluted using Tris buffer containing 1% BSA, and the antibody titers which bound to the antigen in the respective ion intensity buffer solutions under the respective circumstances were obtained based on the produced calibration curve (0 to 100 U/ml).

A difference between the antibody titer at the critical point in each sample thus obtained and the maximum antibody titer at ion intensities lower than the critical point was derived, and antibodies which bound to the double-stranded DNA through electrostatic affinity were detected.

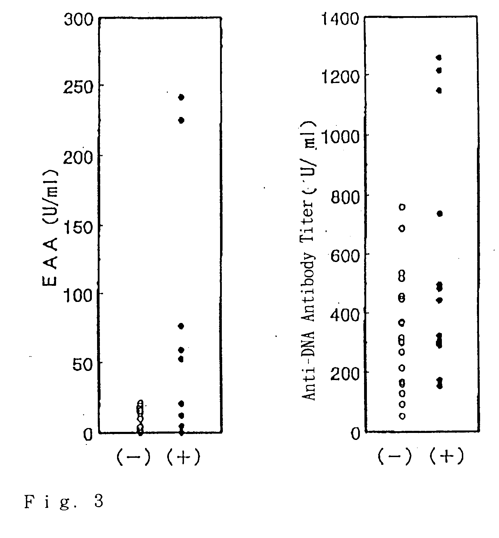

The results obtained are shown in Figure **3** in comparison with those of a conventional method. The anti-DNA antibody titers used for the conventional method were obtained by "Recombigen ELISA anti-ds DNA kit" commercially available from Nihon DPC.

As shown in Figure **3**, the EAA by the measurement method of the present invention provided an evaluation with closer correlation with pathosis than do the anti-DNA antibody titers of the conventional method.

### Industrial Applicability

The method of measuring autoantibodies according to the present invention enables a specific and easy measurement of autoantibodies binding to an antigen through electrostatic affinity, which are considered to be closely related to the pathosis of SLE, and a more accurate diagnosis of SLE.

## Claims

1. A method for measuring an autoantibody comprising the steps of:
allowing a humor to react with a negatively charged substance in at least three kinds of buffer solutions having different ion intensities so as to measure the binding amount of the autoantibody included in the humor to the negatively charged substance in each buffer solution;
and determining the amount of the autoantibody binding to the negatively charged substance by evaluating a difference between the minimum value of binding amounts and the maximum value of binding amounts at ion intensities lower than the ion intensity corresponding to the minimum value.

2. A method according to claim 1, wherein the ion intensities of the buffer solutions are about 0.50 or less.

3. A method according to claim 1, wherein the difference between the ion intensities of the buffer solutions is about 0.01 to about 0.50.

4. A method according to claim 1, wherein the humor is blood, plasma, serum, ascites, lymph, intra-articular fluid, cerebrospinal fluid, or a fraction component obtained therefrom.

5. A method according to claim 1, wherein the negatively charged substance is DNA or cardiolipin.

6. A method according to claim 1, wherein the negatively charged substance is a compound containing one, two, or more of a sulfate ester group, a sulfonic acid group, a carboxyl group, or a phosphate ester group.

7. A method according to claim 1, wherein a salt used for the buffer solutions is NaCl, KCl, or CaCl₂.

8. A method according to claim 1, wherein the measurement of the binding amount is conducted by enzyme immunoassay.

9. A measurement kit for performing the measurement method according to claim 1, the kit containing at least three kinds of buffer solutions having different ion intensities.

10. A method for diagnosing systemic lupus erythematosus comprising the steps of:
allowing a humor to react with a negatively charged substance in at least three kinds of buffer solutions having different ion intensities so as to measure the binding amount of an autoantibody included in the humor to the negatively charged substance in each buffer solution;
and evaluating the amount of the autoantibody binding to the negatively charged substance by determining a difference between the minimum value of binding amounts and the maximum value of binding amounts at ion intensities lower than the ion intensity corresponding to the minimum value.

11. A use of at least three kinds of buffer solutions having different ion intensities and a negatively charged substance for measuring an autoantibody.

12. A use of at least three kinds of buffer solutions having different ion intensities and a negatively charged substance for diagnosing systemic lupus erythematosus.
